Europäisches Patentamt

European Patent Office

Office européen des brevets

(19)

(11) **EP 1 124 604 B1**

(12) **EUROPÄISCHE PATENTSCHRIFT**

(45) Veröffentlichungstag und Bekanntmachung des Hinweises auf die Patenterteilung:
**23.03.2005 Patentblatt 2005/12**

(21) Anmeldenummer: **99970929.8**

(22) Anmeldetag: **27.10.1999**

(51) Int Cl.⁷: $A61M\ 16/00$

(86) Internationale Anmeldenummer:
**PCT/EP1999/008130**

(87) Internationale Veröffentlichungsnummer:
**WO 2000/024446 (04.05.2000 Gazette 2000/18)**

(54) **VORRICHTUNG ZUR BEURTEILUNG DES ANLIEGENDEN LUFTDRUCKS BEI DER AUTOMATISIERTEN BEATMUNG DURCH POSITIVEN LUFTDRUCK AUF DIE ATEMWEGE**

DEVICE FOR ASSESSING THE AIR PRESSURE BEING APPLIED IN AUTOMATIC VENTILATION THROUGH POSITIVE AIRWAY PRESSURE

DISPOSITIF PERMETTANT D'EVALUER LA PRESSION DE L'AIR APPLIQUEE AUX VOIES RESPIRATOIRES DURANT LA VENTILATION EN PRESSION POSITIVE

(84) Benannte Vertragsstaaten:
**AT BE CH CY DE DK ES FI FR GB GR IE IT LI LU MC NL PT SE**

(30) Priorität: **27.10.1998 DE 19849571**

(43) Veröffentlichungstag der Anmeldung:
**22.08.2001 Patentblatt 2001/34**

(73) Patentinhaber: **MAP Medizintechnik für Arzt und Patient GmbH & Co. KG**
**82152 Martinsried (DE)**

(72) Erfinder:
• **GENGER, Harald**
**D-82319 Starnberg (DE)**

• **DRUMM, Peter**
**D-80689 München (DE)**

(74) Vertreter: **VOSSIUS & PARTNER**
**Siebertstrasse 4**
**81675 München (DE)**

(56) Entgegenhaltungen:
| | |
|---|---|
| EP-A- 0 651 971 | EP-A- 0 671 180 |
| EP-A- 0 704 269 | WO-A-95/32016 |
| WO-A-97/22377 | WO-A-98/47554 |
| DE-A- 3 306 607 | GB-A- 2 077 444 |

EP 1 124 604 B1

**Beschreibung**

Die Erfindung betrifft eine Vorrichtung zur Beurteilung des anliegenden Luftdrucks bei der automatisierten Beatmung durch positiven Luftdruck auf die Atemwege. Die Vorrichtung kann in der CPAP (Continuous Positive Airway Pressure-) Therapie zum Einsatz kommen. Die CPAP-Therapie wird in Chest. Vol. 110, Seiten 1077 bis 1088, Oktober 1996 und Sleep, Vol. No. 19, Seiten 184 bis 188 näher beschrieben.

In der CPAP-Therapie wird einem Patienten z.B. in der Nacht ein konstanter positiver Druck über eine Nasenmaske zugeführt. Dieser Überdruck soll gewährleisten, daß die oberen Atemwege während der gesamten Nacht vollständig geöffnet bleiben und somit keine obstruktiven Atmungsstörungen auftreten. Da sich der dazu erforderliche Druck während der Nacht je nach dem Schlafstadium und der Körperposition ändern kann, muß entweder ein variabler Druck oder der größte erforderliche Druck der während der Nacht benötigt wird, dem Patienten zugeführt werden. Vorteilhaft ist es, wenn dem Patienten ein optimaler Druck, zugeführt werden kann. Unter einem optimalen Luftdruck $P_{opt}$ oder auch effektiven Luftdruck $P_{effektiv}$ ist der Druck zu verstehen, bei dem normaler Atemgasfluß zum Patienten vorliegt und eine Steigerung des Druckes nicht zu einer Erhöhung des Atemgasflusses führt.

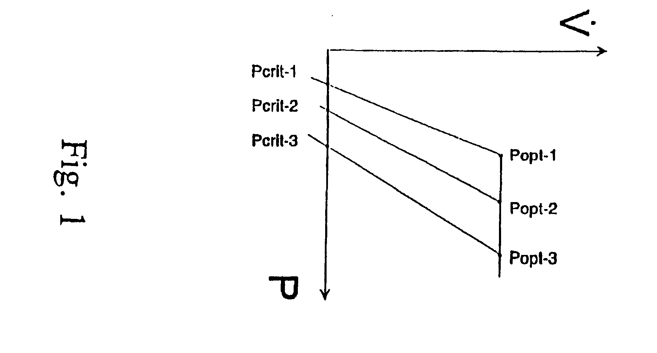

Fig. 1 zeigt, daß der optimale Luftdruck der Knickpunkt der Kurve des Atemgasflusses $\dot{V}$ in Abhängigkeit vom Druck ist.

Die erfindungsgemäße Vorrichtung kann bei einem Therapiegerät (AutoSet) zum Einsatz kommen, bei dem ein maximaler Beatmungsdruck sich automatisch einstellt. Dieses Gerät ist zur Therapieeinstellung in der Klinik bestimmt.

Eine weitere Anwendungsmöglichkeit der erfindungsgemäßen Vorrichtung ist ein Therapiegerät (AutoCPAP), bei dem während der Nacht der Luftdruck an die unterschiedlichen Bedürfnisse des Patienten angepaßt wird. Bei diesem Gerät wird der optimale Druck entsprechend den Bedürfnissen des Patienten laufend neu eingestellt. Das heißt, der Druck wird erhöht, beibehalten oder erniedrigt.

Bei einem Gerät mit einem Algorithmus für die Einstellung bzw. Anpassung des positiven Luftdrucks in der CPAP-Therapie kann ein initialer Beatmungsdruck mit einem Schwellenwert für den Atemgasfluß verglichen werden. In dem Gerät wird der kritische Druck $P_{crit}$ und der Atemwegswiderstand berechnet, und danach der Sollwert des positiven Luftdrucks so gesteuert, daß er entweder beibehalten wird oder entsprechend geändert wird. WO95/32016 A1 zeigt die Merkmale des Preambles des Anspruchs 1.

Der Erfindung liegt die Aufgabe zugrunde, eine Vorrichtung zur Beurteilung des anliegenden Luftdrucks bei der automatisierten Beatmung durch positiven Luftdruck auf die Atemwege zur Verfügung zu stellen, mit der festgestellt werden kann, ob der anliegende Druck der optimale Luftdruck ist oder von ihm abweicht.

Zur Lösung der Aufgabe geht die Erfindung von dem Grundgedanken aus, den anliegenden positiven Luftdruck durch eine Einstell-Einrichtung zu variieren, so daß mindestens drei unterschiedliche Druckwerte eingestellt werden, und mittels einer Meß-Einrichtung den Atemgasfluß für die drei unterschiedlichen Druckwerte zu messen. Aus der Relation der Atemgasflußwerte zueinander wird durch eine Beurteilungs-Einrichtung beurteilt, ob der anliegende Luftdruck unterhalb oder oberhalb des optimalen Luftdrucks liegt oder ob er der optimale Luftdruck ist. Diese Aufgabe wird erfindungsgemäss von der Vorrichtung gemäss Anspruch 1 gelöst.

Vorteilhafterweise kann durch die Erfindung schon nach wenigen Messungen, d.h. ohne unnötige Belastung des Patienten festgestellt werden, in welchem Bereich der anliegende Luftdruck liegt, so daß dieser bei einer eventuellen Abweichung auf den optimalen Luftdruck eingestellt werden kann.

Im folgenden wird die Erfindung anhand der Zeichnungen höher erläutert. Es zeigen:

Fig. 1    ein Gasfluß/Druckdiagramm, das die Änderung der Gasflußkurve während der Nachtzeit zeigt,

Fig. 2    ein Diagramm, das den Druck und den Gasfluß während einer Diagnose und Therapiephase am Patienten anzeigt, und

Fig. 3a-c  Gasfluß/Druck-Diagramme mit Atemgasflußwerten, die Abflußwerte, die unterhalb, im Bereich, bzw. oberhalb des optimalen Drucks liegen.

Fig. 1 zeigt drei mögliche Atemgasflußkurven, die sich während der Nacht am Patienten einstellen können. Je nach Schlafstadium und Körperposition kann der kritische Druck, der die oberen Atemwege vor dem Verschluß bewahrt ($P_{crit-1}$ bis $P_{crit-3}$) sich verändern und zu unterschiedlichen Werten für den optimalen Druck ($P_{opt-1}$ bis $P_{opt-3}$) führen. Die Atemgasflußkurven können sich während der Nacht mehrfach ändern.

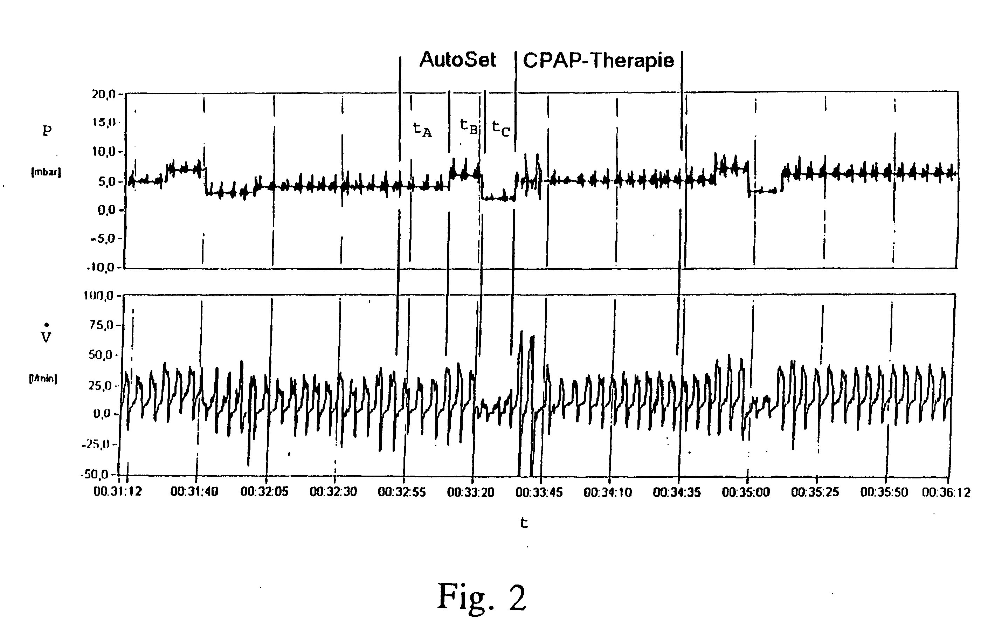

Mittels einer in Fig. 2 dargestellten Folge von Diagnose und Therapiephasen während der Beatmung wird mit der erfindungsgemäßen Vorrichtung ständig beurteilt, ob der angelegte Luftdruck der optimale Luftdruck ist, d.h. sich im Bereich um die Knickpunkte der Gasflußkurven in Fig. 1 befindet oder nicht, und ob der Druck während der Therapiephase aufrechterhalten oder angepaßt werden muß. Wie dem Druckverlauf im oberen Teil von Fig. 2 zu entnehmen ist, wird zu Beginn der Diagnosephase der Druck P für eine bestimmte Anzahl von Atemzügen beibehalten und der Gasfluß $\dot{V}$ (unterer Teil von Fig. 2) gemessen, wobei während der Einatmungsphase Gasflußmaxima auftreten.

Danach wird während der gleichen Anzahl von Atemzügen wie während der Periode $t_A$ der Druck während der Periode $t_B$ erhöht und der Gasfluß gemessen. Daran schließt sich die Periode $t_C$ an, in der der Druck bezüglich des Eingangsdruckes (A) abgesenkt wird und während der gleichen Anzahl von Atemzügen wie unter A der Gasfluß gemessen wird. In der an die Diagnosephase anschließende Therapiephase wird die Beatmung entsprechend der Ergebnisse der Diagnosephase entweder bei unveränderten, oder bei erhöhten bzw. erniedrigtem Druck fortgesetzt.

In einer bevorzugten erfindungsgemäßen Ausführungsform werden die Maxima der gemessenen Gasflußwerte innerhalb jedes Diagnosebereichs gemittelt und die zu den Druckwerten A bis C gehörenden Gasflußwerte $\dot{V}_A$ bis $\dot{V}_C$ bestimmt.

In einer ersten erfindungsgemäßen Ausführungsform erfolgt die Beurteilung des anliegenden Luftdrucks durch Einordnen der Gasflußwerte in das Gasfluß/Druckdiagramm mittels Größenvergleich.

Wenn die Beziehung gilt:

$$k \cdot \dot{V}_B > \dot{V}_A \text{ und } k \cdot \dot{V}_A > \dot{V}_C$$

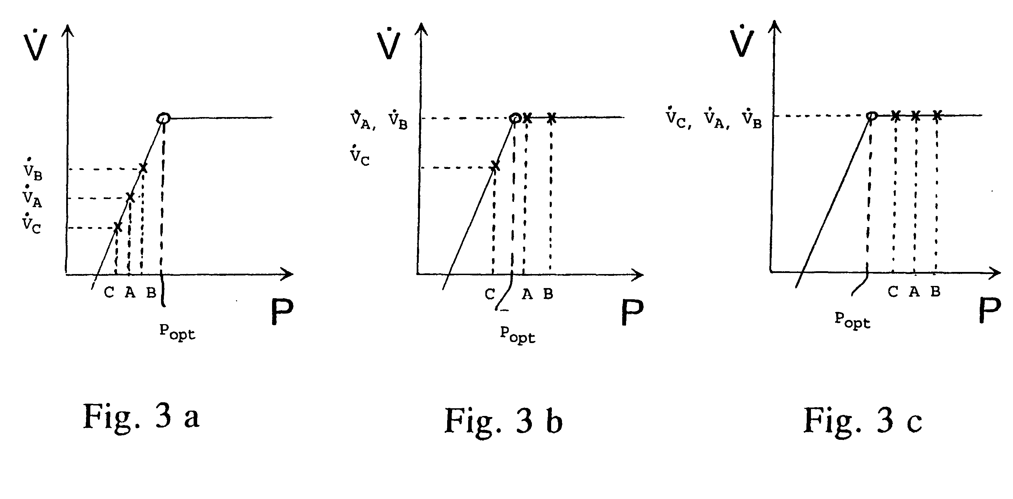

(k < 1,0, vorzugsweise: $0,8 \leq k \leq 0,95$; besonders bevorzugt k = 0,9)
beurteilt die Vorrichtung, daß der Druck P unterhalb des optimalen Drucks $P_{opt}$ liegt. Dieser Sachverhalt ist in Fig. 3a dargestellt.

Wenn gilt:

$$k \cdot \dot{V}_B < \dot{V}_A \text{ und } k \cdot \dot{V}_A < \dot{V}_C$$

dann beurteilt die Vorrichtung, daß der angelegte Druck P oberhalb des optimalen Drucks $P_{opt}$ liegt. Dieser Sachverhalt ist in Fig. 3c dargestellt.

In allen anderen Fällen, d.h. wenn die Gasflußwerte nicht den vorstehend genannten Bedingungen entsprechen, beurteilt die Vorrichtung, daß der angelegte Druck P im Bereich des optimalen Drucks $P_{opt}$ liegt. Ein solcher Fall ist in Fig. 3b dargestellt. Für die Gasflußwerte in diesem Diagramm gilt $k \dot{V}_B < \dot{V}_A$, jedoch ist $k \cdot \dot{V}_A > \dot{V}_C$.

Das entspricht den Verhältnissen um den optimalen Druck im Gasfluß/Druckdiagramm, wobei die Gasflußwerte um den optimalen Druck herum mit sinkendem Druck abfallen.

In einer zweiten erfindungsgemäßen Ausführungsform erfolgt die Auswertung der Maxima des Gasflusses in der Diagnosephase mittels der Regressionsmethode. Dieses Verfahren ist z.B. in "Statistik" J. Hartung, Oldenbourgverlag, 8. Auflage, Seiten 573 bis 581 beschrieben.

Durch die Menge der Gasflußmaxima wird eine Regressionsgerade mit den Parametern $a_{reg}$ (Absolutglied) und $b_{reg}$ (Steigung) gelegt. Da die Varianzen dieser Parametern von zufälligen Fehlern abhängen, können die Varianzen geschätzt werden. Das aus der geschätzten Varianz berechnete Konfidenzintervall für einen Parameter gibt den Bereich an, in dem sich der Parameter mit der Wahrscheinlichkeit $\gamma$ bewegt. Für die Beurteilung, ob die Regressionsgerade mit einer gewissen Wahrscheinlichkeit eine positive Steigung besitzt, sollen beide Grenzen des Konfidenzintervalls für den Parameter $b_{reg}$ größer 0 sein und bei einer negativen Steigung kleiner Null sein. Haben die beiden Intervallgrenzen unterschiedliche Vorzeichen, so kann keine eindeutige Aussage über die Steigung der Geraden getroffen werden.

Für die Beurteilung des anliegenden Drucks bei der automatisierten Beatmung ergeben sich daraus folgende Fälle.

- Beide Grenzen des Konfidenzintervalls sind > 0:
  Die Gasflußmaxima liegen mit großer Wahrscheinlichkeit auf einer Geraden mit positiver Steigung, also auf dem ansteigenden Teil des Gasfluß/Druckdiagramms. Das bedeutet, daß der Druck als unterhalb des optimalen Drucks liegens beurteilt wird.

- Beide Grenzen des Konfidenzintervalls sind < 0:
  Wird ein Patient mit einem zu hohen CPAP-Druck behandelt, so hat man beobachtet, daß seine Atemanstrengung ansteigt und damit der Gasfluß abnimmt. Erniedrigt man den Druck, so wird sich der Gasfluß wieder erhöhen. Daraus folgt, daß bei einer Geraden mit negativen Steigung der anliegende Druck als oberhalb des optimalen Drucks liegend beurteilt wird

- Die Grenzen des Konfidenzintervalls haben unterschiedliche Vorzeichen.
  Da die Gerade keine eindeutige Positiv- oder Negativsteigung aufweist, wird angenommen, daß sich der Gasfluß mit zunehmenden Druck nicht mehr wesentlich geändert hat. Eine weitere Erhöhung des Drucks würde also keine Verbesserung des Gasflusses mit sich bringen. Es wird der anliegende Druck als der optimale Druck beurteilt.

In folgenden wird die Berechnung der Regressionsgeraden, des Schätzwertes für die Fehlervarianz der Steigung der Regressionsgeraden und des Konfidenzintervalls angegeben.

Bestimmung der Regressionsgeraden nach dem Kriterium des kleinsten quadratischen Fehlers:

$$Y_{reg} = b_{reg} \cdot X + a_{reg}$$

mit

$$b_{reg} = \Sigma(X_i - X_{mean})(Y_i - Y_{mean})/\Sigma(X_i - X_{mean})^2 \qquad (1)$$

$$a_{reg} = Y_{mean} - b_{reg} \cdot X_{mean}$$

Berechnung des Schätzwertes für die Fehlervarianz der Steigung der Regressionsgeraden:

$$s_b^2 = s^2/\Sigma(X_i - X_{mean})^2 \text{ mit: } s^2 = 1/(n-2) \cdot \Sigma(Y_i - Y_{reg,i})^2 \qquad (2)$$

Konfidenzintervall:

$$[b_{reg} - s_b \cdot t_{n-2,\ 1-\gamma/2};\ b_{reg} + s_b \cdot t_{n-2,\ 1-\gamma/2}] \qquad (3)$$

$t_{n-2:\ 1-\gamma/2}$ Tabellenwerte für $\gamma = 0{,}975$.

Die erfindungsgemäße Vorrichtung kann zum Einstellen des optimalen Luftdrucks $P_{opt}$ bei eimem Beatmungsgerät, bei dem ein maximal erforderlicher Behandlungsdruck eingestellt wird, verwendet werden. Ein solches Beatmungsgerät, das z.B. unter dem Namen AutoSet bekannt ist, wird in der Klinik zur Therapieeinstellung eines Patienten verwandt. Mittels der erfindungsgemäßen Vorrichtung wird der maximale optimale Druck $P_{opt}$ bestimmt, dem entspricht in Fig. 1 der Punkt $P_{opt-3}$. Dieser Punkt wird durch Druckerhöhung eingestellt, wenn die Vorrichtung beurteilt, daß der anliegende Druck unterhalb des optimalen Drucks liegt, oder durch Beibehalten des anliegenden Drucks wenn die Vorrichtung bestimmt, daß der anliegende Druck im Bereich des optimalen Drucks liegt. Die Beurteilung, daß der anliegende Druck oberhalb des optimalen Drucks liegt, führt bei diesem Gerät nicht zum Erniedrigen des anliegenden Drucks.

Die erfindungsgemäße Vorrichtung kann auch bei einem Beatmungsgerät verwendet werden, das unter der Bezeichnung AutoCPAP bekannt ist, welches während der Nacht den Therapiedruck an die Bedürfnisse des Patienten anpaßt und dabei den optimalen Druck $P_{opt}$ auch erniedrigt. Dabei wird auch das Beurteilungsergebnis der Vorrichtung, daß der anliegende Druck oberhalb des optimalen Drucks liegt, herangezogen, um den anliegenden Druck abzusenken. In Figur 1 bedeutet das, daß sich während der Nacht der anliegende Druck zwischen den Werten $P_{opt-1}$ und $P_{opt-3}$ bewegt. Damit ist gewährleistet, daß der Patient immer ausreichend beatmet wird und nie einen zu hohen Druck zugeführt bekommt.

Zum Ausschalten von technischen Fehlern kann die Reaktion auf die Beurteilung durch die erfindungsgemäße Vorrichtung zeitlich verzögert werden. Das bedeutet, daß mehrere, bevorzugt drei gleiche Analyseergebnisse abgewartet werden, bevor eine Veränderung des anliegenden Drucks vorgenommen wird.

Zur Überwachung des Zustands eines Patienten, z.B. durch medizinisches Personal, kann das Beurteilungsergebnis auf einer Anzeigeeinrichtung sichtbar gemacht werden oder ein akustisches Signal auslösen.

**Patentansprüche**

1. Vorrichtung zur Beurteilung des anliegenden Luftdrucks (P) bei der automatisierten Beatmung durch positiven Luftdruck auf die Atemwege, mit
   einer Einstell-Einrichtung zum Variieren des anliegenden Luftdrucks (P), so dass mindestens drei unterschiedliche Druckwerte (A, B, C) eingestellt werden,

einer Meßeinrichtung zum Messen des Atemgasflusses ($\dot{V}$) für die drei unterschiedlichen Druckwerte (A, B, C), wobei drei entsprechende Atemgasflußwerte ($\dot{V}_A$, $\dot{V}_B\dot{V}_C$) erhalten werden, und

einer Beurteilungs-Einrichtung zum Beurteilen aus der Relation der gemessenen Atemgasflußwerte ($\dot{V}_A$, $\dot{V}_B$, $\dot{V}_C$) zueinander, ob der anliegende Luftdruck (P) unterhalb oder oberhalb des optimalen Luftdrucks ($P_{opt}$) liegt oder ob er der optimale Luftdruck ($P_{opt}$) ist, **dadurch gekennzeichnet, dass** die Vorrichtung angepaßt ist, in den folgenden Schritten zu arbeiten:

(a) Messen des Atemgasflusses ($\dot{V}_A$) durch die Meß-Einrichtung während einer bestimmten Anzahl von Atemzügen unter Beibehaltung des Wertes A,

(b) Erhöhen des Luftdrucks (P) durch die Einstell-Einrichtung auf den Wert B und Messen des Atemgasflusses ($\dot{V}_B$) während der gleichen Anzahl von Atemzügen wie in Schritt (a), und

(c) Erniedrigen des Luftdrucks (P) durch die Einstell-Einrichtung auf den Wert C und Messen des Atemgasflusses ($\dot{V}_C$) während der gleichen Anzahl von Atemzügen wie in Schritt (a).

**2.** Vorrichtung nach Anspruch 1, wobei die Beurteilungs-Einrichtung angepaßt ist,

(a) den Druck (P) als unterhalb des optimalen Drucks ($P_{opt}$) liegend zu beurteilen, wenn gilt:

$$k \cdot \dot{V}_B > \dot{V}_A \text{ und } k \cdot \dot{V}_A > \dot{V}_C$$

(b) den Druck P als oberhalb des optimalen ($P_{opt}$) liegend zu beurteilen, wenn gilt:

$$k \cdot \dot{V}_B < \dot{V}_A \text{ und } k \cdot \dot{V}_A < \dot{V}_C \, ,$$

und

(c) bei allen anderen Werten der Atemgasflußkurve ($\dot{V}$) den Druck (P) als den optimalen Druck ($P_{opt}$) zu beurteilen,

wobei k < 1,0 ist, vorzugsweise $0{,}8 \leq k \leq 0{,}95$, besonders bevorzugt k = 0,9, und die Druckwerte folgende Beziehung erfüllen: C < A < B.

**3.** Vorrichtung nach Anspruch 1, wobei die Beurteilungs-Einrichtung angepaßt ist, um die Relation der Atemgasflußwerte ($\dot{V}_A$, $\dot{V}_B$, $\dot{V}_C$) mittels der Regressionsanalyse zu gewinnen.

**4.** Vorrichtung nach Anspruch 3, wobei die Beurteilungs-Einrichtung angepaßt ist,

(a) die Regressionsgerade durch die Atemgasflußwerte ($\dot{V}_A$, $\dot{V}_B$, $\dot{V}_C$) zu berechnen,

(b) das Konfidenzintervall der Steigung der Regressionsgeraden an der Stelle eines der eingestellten Druckwerte (A, B, C) zu berechnen,

($c_1$) den Druck (P) als unterhalb des optimalen Drucks ($P_{opt}$) liegend zu beurteilen, wenn beide Grenzen des Konfidenzintervalls > 0 sind,

($c_2$) den Druck (P) als oberhalb des optimalen Drucks ($P_{opt}$) liegend zu beurteilen, wenn beide Grenzen des Konfidenzintervalls < 0 sind, und

($c_3$) den Druck (P) als den optimalen Druck ($P_{opt}$) zu beurteilen, wenn die Grenzen des Konfidenzintervalls unterschiedliche Vorzeichen aufweisen.

**5.** Vorrichtung nach einem der Ansprüche 1 bis 4, wobei die Einstell-Einrichtung angepaßt ist, als Druckwert A 4 mbar, als Druckwert B 6 mbar und als Druckwert C 2 mbar einzustellen.

**6.** Vorrichtung nach einem der Ansprüche 1 bis 5, wobei die Meß-Einrichtung angepaßt ist, den Atemgasfluß ($\dot{V}_A$, $\dot{V}_B$, $\dot{V}_C$) durch Messen der Maxima in der Einatmungsphase und Bildung ihres arithmetischen Mittels zu bestimmen.

**Claims**

**1.** A device for assessing the applied air pressure (P) during automatic respiration through positive airway pressure comprising

an adjusting means for varying the applied pressure (P) so that at least three different pressure values (A, B, C) can be adjusted,
a measuring means for measuring the respiratory flow ($\dot{V}$) for the three different pressure values (A, B, C), wherein three corresponding respiratory gas flow values ($\dot{V}_A$, $\dot{V}_B$, $\dot{V}_C$) are obtained, and
an assessing means for assessing on the basis of the relation of the measured respiratory gas flow values ($\dot{V}_A$, $\dot{V}_B$, $\dot{V}_C$) to each other whether the applied air pressure (P) lies below or above the optimal air pressure ($P_{opt}$) or whether it is the optimal air pressure ($P_{opt}$), **characterized in that** said device is adapted to operate in the following steps:

(a) measuring the respiratory gas flow ($\dot{V}_A$) by means of the measuring means during a predetermined number of breaths, thereby maintaining the value A,

(b) increasing the air pressure (P) by means of the adjusting means to the value B and measuring the respiratory gas flow ($\dot{V}_B$) during the same number of breaths as in step (a), and

(c) decreasing the air pressure (P) by means of the adjusting means to the value C and measuring the respiratory gas flow ($\dot{V}_C$) during the same number of breaths as in step (a).

2. The device according to claim 1, wherein the assessing means is adapted

(a) to assess that the pressure (P) lies below the optimal pressure ($P_{opt}$) if it is true that

$$k \cdot \dot{V}_B > \dot{V}_A \text{ and } k \cdot \dot{V}_A > \dot{V}_C$$

(b) to assess that the pressure (P) lies above the optimal pressure ($P_{opt}$) if it is true that

$$k \cdot \dot{V}_B < \dot{V}_A \text{ and } k \cdot \dot{V}_A < \dot{V}_C$$

and
(c) in case of all other values of the respiratory gas flow curve ($\dot{V}$), to assess that the pressure (P) is the optimal pressure ($P_{opt}$),

wherein $k < 1,0$, preferably $0.8 \leq k \leq 0.95$, particularly preferably $k = 0.9$ and
wherein the pressure values fulfil the following condition: $C < A < B$.

3. The device according to claim 1, wherein the assessing means is adapted to assess the relation of the respiratory gas flow values ($\dot{V}_A$, $\dot{V}_B$, $\dot{V}_C$) by means of the regression analysis.

4. The device according to claim 3, wherein the assessing means is adapted

(a) to calculate the regression straight-line on the basis of the respiratory gas flow values ($\dot{V}_A$, $\dot{V}_B$, $\dot{V}_C$),

(b) to calculate the confidence interval of the gradient of the regression straight-line at the position of one of the adjusted pressure values (A, B, C),

($c_1$) to assess that the pressure (P) lies below the optimal pressure ($P_{opt}$) if both limits of the confidence interval are $> 0$,

($c_2$) to assess that the pressure (P) lies above the optimal pressure ($P_{opt}$) if both limits of the confidence interval are $< 0$, and

($c_3$) to assess that the pressure (P) is the optimal pressure ($P_{opt}$) if the limits of the confidence interval have different signs.

5. The device according to any one of claims 1 to 4, wherein the adjusting means is adapted to adjust 4 mbar as pressure value A, 6 mbar as pressure value B and 2 mbar as pressure value C.

**6.** The device according to any one of claims 1 to 5, wherein the measuring means is adapted to determine the respiratory gas flow ($\dot{V}_A$, $\dot{V}_B$, $\dot{V}_C$) by measuring the maxima in the inhalation phase and formation of their arithmetic mean value.

**Revendications**

**1.** Dispositif permettant d'évaluer la pression de l'air (P) appliquée aux voies respiratoires durant la ventilation automatisée en pression positive, comportant un dispositif de réglage pour varier la pression de l'air (P) appliquée de sorte qu'au moins trois différentes valeurs de pression (A, B, C) peuvent être réglées,
un dispositif de mesure permettant de mesurer le flux de gaz respiratoire ($\dot{V}$) pour les trois différentes valeurs de pression (A, B, C), les trois valeurs de flux de gaz respiratoire ($\dot{V}_A$, $\dot{V}_B$, $\dot{V}_C$) correspondantes étant obtenues et un dispositif d'évaluation permettant d'évaluer à partir de la relation des valeurs de flux de gaz respiratoire ($\dot{V}_A$, $\dot{V}_B$, $\dot{V}_C$) mesurées entre elles, si la pression de l'air appliquée (P) se situe au-dessus ou au-dessous de la pression de l'air optimale ($P_{opt}$) ou si elle est la pression de l'air optimale ($P_{opt}$), **caractérisé en ce que** le dispositif est adapté pour travailler dans les étapes suivantes :

(a) mesure du flux de gaz respiratoire ($\dot{V}_A$) par le dispositif de mesure pendant un nombre déterminé de respirations, la valeur A étant maintenue,

(b) augmentation de la pression de l'air (P) à la valeur B au moyen du dispositif de réglage et mesure du flux de gaz respiratoire ($\dot{V}_B$) pendant le même nombre de respirations que dans l'étape (a), et

(c) abaissement de la pression de l'air (P) à la valeur C au moyen du dispositif de réglage et mesure du flux dé gaz respiratoire ($\dot{V}_C$) pendant le même nombre dé respirations que dans l'étape (a).

**2.** Dispositif selon la revendication 1, le dispositif d'évaluation étant adapté

(a) pour évaluer la pression (P) comme étant inférieure à la pression optimale ($P_{opt}$) lorsque

$$k \cdot \dot{V}_B > \dot{V}_A \text{ et } k \cdot \dot{V}_A > \dot{V}_C$$

(b) pour évaluer la pression P comme étant au-dessus de la valeur optimale ($P_{opt}$) lorsque

$$k \cdot \dot{V}_B < \dot{V}_A \text{ et } k \cdot \dot{V}_A < \dot{V}_C,$$

et

(c) pour évaluer la pression (P) comme étant la pression optimale ($P_{opt}$) pour toutes les autres valeurs de la courbe de flux de gaz respiratoire ($\dot{V}$),
k étant < 1,0, de préférence $0,8 \leq k \leq 0,95$, tout particulièrement k = 0,9 et les valeurs de pression réalisant la relation suivante: C < A < B.

**3.** Dispositif selon la revendication 1, le dispositif d'évaluation étant adapté pour obtenir la relation des valeurs de flux de gaz respiratoire ($\dot{V}_A$, $\dot{V}_B$, $\dot{V}_C$) au moyen de l'analyse par régression.

**4.** Dispositif selon la revendication 3, le dispositif d'évaluation étant adapté

(a) pour calculer la ligne de régression à travers les valeurs de flux de gaz respiratoire ($\dot{V}_A$, $\dot{V}_B$, $\dot{V}_C$),

(b) pour calculer l'intervalle de confiance de la pente de la ligne de régression au point d'une des valeurs réglées (A, B, C),

($c_1$) pour évaluer la pression (P) comme étant au-dessous de la pression optimale ($P_{opt}$) lorsque les deux limites de l'intervalle de confiance sont > 0,

(c$_2$) pour évaluer la pression (P) comme étant au-dessus de la pression optimale (P$_{opt}$) lorsque les deux limites de l'intervalle de confiance sont < 0, et

(c$_3$) pour évaluer la pression (P) comme étant la pression optimale (P$_{opt}$) lorsque les limites de l'intervalle de confiance présentent des signes différents.

5. Dispositif selon l'une quelconque des revendications 1 à 4, le dispositif de réglage étant adapté pour régler la valeur de pression A à 4 mbar, la valeur de pression B à 6 mbar et la valeur de pression C à 2 mbar.

6. Dispositif selon l'une quelconque des revendications 1 à 5, le dispositif de réglage étant adapté pour déterminer le flux de gaz respiratoire ($\dot{V}_A$, $\dot{V}_B$, $\dot{V}_C$) par la mesure des maxima dans la phase d'inspiration et la formation de leur moyenne arithmétique.

Fig. 1

AutoSet | CPAP-Therapie

Fig. 2

Fig. 3 a          Fig. 3 b          Fig. 3 c

EP 1 124 604 B1